# EUROPEAN PATENT APPLICATION

(11) **EP 4 509 077 A2**
(43) Date of publication of application: **19.02.2025**
(21) Application number: 24194536.9
(22) Date of filing: 14.08.2024
(51) Int. Cl.: A61B 17/74

(54) **RECON HIP SCREW**

(30) Priority: 14.08.2023 US 202363532502 P
(71) Applicant: Stryker European Operations Limited, Carrigtwohill, Co. Cork T45 HX08 (IE)
(72) Inventor: SIMON, Bernd, 24107 Kiel (DE); TERRILL, Lance N., Rochestown, T12Y22H (IE); DURHAM, James, Ballingcollig, P31 XA 38 (IE)
(74) Representative: Maiwald GmbH

(57) **Abstract**

A fracture fixation system includes a fixation element having a plate and a barrel. The plate has an outer surface and an inner surface for placement against an exterior surface of a bone, and the barrel extends along a barrel axis and has a peripheral wall protruding from the inner surface of the plate. The fixation element defines first and second passages through the plate and the barrel that each extend along the barrel axis. The fracture fixation system further includes a first lag screw extending from a proximal end to a distal end and configured to be inserted through the first passage defined by the plate and the barrel. The fracture fixation system further includes a second lag screw extending from a proximal end to a distal end and configured to be inserted through the second passaged defined by the plate and the barrel.

## Description

### CROSS-REFERENCE TO RELATED APPLICATIONS

The present application claims the benefit of the filing date of U.S. Provisional Application No. 63/532,502, filed August 14, 2023, the disclosure of which is hereby incorporated herein by reference.

### FIELD OF THE INVENTION

The present invention relates to orthopedic surgical devices used to join and promote healing of fractured bone, and more particularly, and not by limitation, devices used to fixate proximal femoral fractures.

### BACKGROUND OF THE INVENTION

The surgical treatment of femoral neck fractures utilizing internal fixation remains challenging, especially for dislocated unstable fractures. There are a variety of devices used to treat fractures of the femur, humerus, tibia, and other long bones. For example, fractures of the femoral neck, head, and intertrochanteric region have been successfully treated with a variety of internal fixation means such as compression screw assemblies. Compression hip and bone screw devices for use in fixating a fractured bone during the healing process have been used for years. It is mainstream practice for surgeons to utilize cannulated compression screws (CCS) or sliding hip screws (SHS) as compression screws in internal fixation systems.

For many surgeons and customers, the utilization of CCS or SHS devices remain the treatment of choice. However, CCS accounts for up to nearly 30% of hip fracture failures and their disadvantages are well documented. In particular, CCS devices are not angularly stable, have insufficient rotation control, and suffer from uncontrolled shortening of the femoral neck and limited resistance against shear forces. Disadvantages of SHS are that an additional anti-rotation screw is required with limited space particularly in small anatomies, that they have large lateral footprints, and also that they create a potential collision with a retrograde nail in the case of ipsilateral neck-shaft fixation.

Additionally, problems may result from weakened or poor-quality bone that is adjacent to the fracture site. Often times the bone adj acent to the fracture is weak and is prone to damage when exposed to compression. For example, there could be uncontrolled shortening of the femoral head when the femoral head compresses towards or into the fracture site. In extreme cases, uncontrolled shortening may cause the femoral head to be compressed all the way into the trochanteric region of the femur.

Thus, it would be desirable to provide a fracture fixation system to improve on the prior art disadvantages.

### BRIEF SUMMARY OF THE INVENTION

According to a first aspect of the disclosure, a fracture fixation system may include a fixation element, a first lag screw and a second lag screw. The fixation element may include a plate and a barrel, the plate having an outer surface and an inner surface for placement against an exterior surface of a bone, and the barrel extending along a barrel axis and having a peripheral wall protruding from the inner surface of the plate, the fixation element defining first and second passages through the plate and the barrel that each extending along the barrel axis. The first lag screw may extend from a proximal end to a distal and may be configured to be inserted through the first passage defined by the plate and the barrel. The second lag screw may extend from a proximal end to a distal end and may be configured to be inserted through the second passage defined by the plate and the barrel.

Further in the fracture fixation system according to the first aspect of the disclosure, a first compression nut may be configured to be coupled to the proximal end of the first lag screw when the first lag screw is disposed within the first passage. The first compression nut may define an internally threaded surface that is configured to be threadably coupled to an externally threaded surface at the proximal end of the first lag screw. The barrel may define a first internal shoulder within the first passage, and a distal end of the first compression nut may be configured to abut the first internal shoulder to limit distal movement of the first compression nut within the first passage. A first cap screw may be configured to be coupled to the internally threaded surface of the first compression nut. A first cap screw may be configured to be at least partially disposed within the first compression nut. An end connector may be configured to be coupled to the first cap screw. An end connector may be configured to be disposed within the first and second passages of the barrel.

Further in the fracture fixation system according to the first aspect of the disclosure, a first compression nut may be configured to be coupled to the first lag screw and a second compression nut may be configured to be coupled to the second lag screw. The first and second compression nuts may each define an internally threaded surface and may be configured to be threadably coupled to proximal portions of each of the first and second lag screws, respectively. The barrel may define a first internal shoulder within the first passage and a second internal shoulder within the second passage, and a distal end of the first compression nut may be configured to abut the first internal shoulder and a distal end of the second compression nut may be configured to abut the second internal shoulder. A first cap screw may be configured to be coupled to the first compression nut and a second cap screw may be configured to be coupled to the second compression nut. The first and second cap screws may each define a proximal lip extending radially outward, and the system may further include an end connector having a pair of distal lips, wherein each of the pair of distal lips is adapted to engage the corresponding proximal lip of the first and second cap screws to couple the end connector to the first and second cap screws.

Further in the fracture fixation system according to the first aspect of the disclosure, the first lag screw and the second lag screw may be identical in size and shape. The first passage may be separated from the second passage by a central portion of the barrel. The first lag screw may include a distal threaded portion having threads oriented in a first rotational direction along a length of the distal threaded portion, and the second lag screw may include a distal threaded portion having threads oriented in a second direction opposite the first rotational direction along a length of the distal threaded portion of the second lag screw. The first lag screw may include a proximal portion having teeth configured to be disposed within the first passage, and the second lag screw may include a proximal portion having teeth configured to be disposed within the second passage. The first passage may be in communication with the second passage. The teeth of the first lag screw may be adapted to engage with the teeth of the second lag screw within the barrel, such that rotation of the first lag screw in a first direction causes rotation of the second lag screw in a second direction opposite the first direction. The teeth of the first lag screw may protrude from the perimeter of the proximal portion of the first lag screw and each tooth may extend longitudinally along the proximal portion of the first lag screw, and the teeth of the second lag screw may protrude from the perimeter of the proximal portion of the second lag screw and each tooth may extend longitudinally along the proximal portion of the second lag screw. A proximal end of the first lag screw may be adapted to receive a compression screw. A proximal end of the second lag screw may be adapted to receive a compression screw. The first lag screw may include a distal threaded portion having threads oriented in a first rotational direction along a length of the distal threaded portion, and the second lag screw may include a distal threaded portion having threads oriented in the first rotational direction along a length of the distal threaded portion of the second lag screw. Each thread of the first lag screw may be configured to be positioned between a pair of adjacent threads on the distal threaded portion of the second lag screw. The first lag screw is adapted to receive a rotation screw to rotate the first lag screw. The second lag screw may be adapted to receive a rotation screw to rotate the second lag screw.

According to a second aspect of the disclosure, a kit may include a fracture fixation element, a first lag screw, a first compression nut, a first cap screw and an end connector. The fixation element may include a plate and a barrel, the plate having an outer surface and an inner surface for placement against an exterior surface of a bone, and the barrel extending along a barrel axis and having a peripheral wall protruding from the inner surface of the plate, the fixation element defining a passage through the plate and the barrel that extends along the barrel axis. The first lag screw may extend from a proximal end to a distal end and may be configured for insertion into the passage defined by the plate and the barrel. The first compression nut may be configured to be disposed within the passage around the first lag screw. The first cap screw may be configured to be coupled to the proximal end of the first lag screw. The end connector may be configured to be coupled to the first cap screw.

Further in the kit according to the second aspect of the disclosure, the compression nut may be configured to threadably couple to the first lag screw, and when the first lag screw is assembled to the fixation element, a distal end of the first compression nut may be configured to abut a first internal shoulder formed in the passage of the fixation element. The kit may further include a second lag screw, a second compression nut and a second cap screw. The second lag screw may extend from a proximal end to a distal end and configured for insertion into the passage defined by the plate and the barrel. The second compression nut may be configured to be disposed within the passage around the second lag screw. The second cap screw may be configured to be coupled to the proximal end of the second lag screw. The end connector may be configured to couple to the second cap screw. The first lag screw may be pre-assembled to the first compression nut and the second lag screw may be pre-assembled to the second compression nut. The first cap screw and the second cap screw may be pre-assembled to the end connector. The first lag screw may be configured for insertion into a first superior portion of the passage and the second lag screw may be configured for insertion into a second inferior portion of the passage. The first and second lag screws may be interchangeable such that the first and second lag screws are configured for insertion into a first superior portion of the passage and the first and second lag screws are configured for insertion into a second inferior portion of the passage.

According to a third aspect of the disclosure, a method of using a fracture fixation system may including the steps of drilling a first superior bore having a first diameter to a first depth through the femoral neck; drilling a first inferior bore having a second diameter to a second depth through the femoral neck; drilling a second superior bore within the first superior bore to a third depth, the second superior bore having a third diameter smaller than the first diameter; drilling a second inferior bore within the first inferior bore to a fourth depth, the second inferior bore having a fourth diameter smaller than the second diameter; mounting a fixation element to the femur, including placing an inner surface of a plate of the fixation element against an exterior surface of the femur, and inserting a peripheral wall of a barrel of the fixation element into the first superior bore and the first inferior bore; inserting a first lag screw into a passage defined through the plate and the barrel of the fixation element such that a distal end of the lag screw extends into the second superior bore; and inserting a second lag screw into the passage defined through the plate and the barrel of the fixation element such that a distal end of the lag screw extends into the second inferior bore.

Further in the method of the third aspect of the disclosure, the method may include threadably coupling a first compression nut to a proximal portion of the first lag screw and threadably coupling a second compression nut to a proximal portion of the second lag screw. The method may include rotating a first compression nut threadably coupled to the first lag screw relative to the first lag screw, and rotating a second compression nut threadably coupled to the second lag screw relative to the second lag screw. Rotating the first compression nut may include translating the first lag screw in a proximal direction relative to the first compression nut, and rotating the second compression nut may include translating the second lag screw in a proximal direction relative to the second compression nut. The method may include threadably connecting a first cap screw the first compression nut and threadably connecting a second cap screw to the second compression nut. The method may include coupling a first cap screw and a second cap screw pre-assembled to an end connector to the first compression nut and the second compression nut, respectively. The method may include inserting a temporary fixator through the plate to affix the fixation element to the exterior surface of the femur and removing the temporary fixator from the plate.

Further in the method of the third aspect of the disclosure, drilling the first superior bore may include reaming the first superior bore for the barrel and drilling the second superior bore may include reaming the second superior bore for the first lag screw. Drilling the first inferior bore may include reaming the first inferior bore for the barrel and drilling the second inferior bore may include reaming the second inferior bore for the second lag screw. The first and second lag screws may be inserted through an aiming device positioned adjacent the fixation element. The method may include simultaneously actuating the first lag screw and the second lag screw. The method may include inserting a k-wire through a femoral neck and into a femoral head before the steps of drilling the first superior and first inferior bores. The step of drilling the first superior bore may include drilling the first superior bore over the k-wire. The step of mounting may include guiding the passage of the fixation element over the k-wire. The step of inserting the first lag screw may include guiding a lumen of the first lag screw over the k-wire. The second diameter may be smaller than the first diameter. The first diameter may be equal to the second diameter. The first depth may be equal to the second depth. The third diameter may be equal to the fourth diameter. The method may include inserting a first guide pin into the second superior bore and inserting a second guide pin into the second inferior bore. Inserting the first lag screw may include advancing the first lag screw along the first guide pin, and inserting the second lag screw may include advancing the second lag screw along the second guide pin. The method may include rotating the first lag screw and rotating the second lag screw. Rotating the first lag screw may include inserting a rotation screw into a proximal end of the first lag screw and rotating the rotation screw with a tool, and rotating the second lag screw may include inserting a rotation screw into a proximal end of the second lag screw and rotating the rotation screw with a tool. Rotating the first lag screw may include rotating the rotation screw in a first direction, the method further including removing the rotation screw by rotating the rotation screw in a second direction opposite the first direction. Rotating the second lag screw may include rotating the rotation screw in a first direction, the method further comprising removing the rotation screw by rotating the rotation screw in a second direction opposite the first direction. The first lag screw may be rotated in a first direction and the second lag screw may be rotated in the first direction. The first lag screw may be rotated in a first direction and the second lag screw may be rotated in a second direction opposite the first direction. The first lag screw may include teeth engaging with teeth of the second lag screw such that rotation of the first lag screw in the first direction causes rotation of the second lag screw in the second direction.

According to a fourth aspect of the disclosure, a fracture fixation system may include a fixation element, a bolt and a lag screw. The fixation element may include a plate and a barrel, the plate having an outer surface and an inner surface for placement against an exterior surface of a bone, and the barrel extending along a barrel axis and having a peripheral wall protruding from the inner surface of the plate, the fixation element defining first and second passages through the plate and the barrel that each extend along the barrel axis. The bold may extend from a proximal end to a distal end and may be configured to be inserted through the first passage defined by the plate and the barrel. The lag screw may extend from a proximal end to a distal end and may be configured to be inserted through the second passage defined by the plate and the barrel.

Further in the fracture fixation system according to the fourth aspect of the disclosure, a proximal end of the lag screw may be adapted to receive a rotation screw to rotate the lag screw. The first passage may be separated from the second passage by a center portion of the barrel. A cross-section of the bolt taken perpendicular to a longitudinal axis of the bolt may be non-circular.

According to a fifth aspect of the disclosure, a fracture fixation system may include a fixation element, a first bold and a second bolt. The fixation element may include a plate and a barrel, the plate having an outer surface and an inner surface for placement against an exterior surface of a bone, and the barrel extending along a barrel axis and having a peripheral wall protruding from the inner surface of the plate, the fixation element defining first and second passages through the plate and the barrel that each extend along the barrel axis. The first bolt may extend from a proximal end to a distal end and may be configured to be inserted through at least the first passage defined by the plate and the barrel. The second bolt may extend from a proximal end to a distal end and may be configured to be inserted through the second passage defined by the plate and the barrel.

Further in the fixation system according to the fifth aspect of the disclosure, a distal portion of the first bolt may include a blade extending oblique to a longitudinal axis of the first bolt. The blade may extend perpendicular to the longitudinal axis of the first bolt. In a first rotational position of the first bolt, the blade may extend toward the second bolt. The blade may define a proximal face, and in the first rotational position, the proximal face of the blade may abut or face a distal end of the second bolt. In a second rotational position, the blade may extend from the first bolt in a direction away from the second bolt. In the second rotational position, the blade may extend in a direction opposite the second bolt. The first bolt may be configured to be rotated from the first rotational position to the second rotational position. A proximal end of the first bolt may be adapted to receive a rotation tool. The first bolt may be configured to be inserted through the first passage and a portion of the second passage.

According to a sixth aspect of the disclosure, a method of using a fracture fixation system may include drilling a first superior bore having a first diameter to a first depth through the femoral neck; drilling a first inferior bore having a second diameter to a second depth through the femoral neck; drilling a second superior bore coaxial with the first superior bore to a third depth, the second superior bore having a third diameter smaller than the first diameter; drilling a second inferior bore coaxial with the first inferior bore to a fourth depth, the second inferior bore having a fourth diameter smaller than the second diameter; mounting a fixation element to the femur, including placing an inner surface of a plate of the fixation element against an exterior surface of the femur, and inserting a peripheral wall of a barrel of the fixation element into the first superior bore and the first inferior bore; inserting a first bolt into a passage defined through the plate and the barrel of the fixation element such that a distal end of the first bolt extends into the second superior bore; and inserting a second bolt into the passage defined through the plate and the barrel of the fixation element such that a distal end of the second bolt extends into the second inferior bore.

Further in the method according to the sixth aspect of the disclosure, the method may include inserting a first guide pin into the second superior bore. Inserting the first bolt may include advancing the first bolt along the first guide pin. The method may include rotating the first bolt to transition the first bolt from a first unanchored position to a second anchored position by repositioning a blade protruding from the first bold within the femur. The first bolt may be rotated by inserting a rotation tool into a proximal end of the first bolt and rotating the rotation tool. Inserting the rotation tool includes advancing the rotation tool along the first guide pin.

### BRIEF DESCRIPTION OF THE DRAWINGS

FIG. 1 is a perspective view of a fracture fixation system according to an embodiment of the disclosure.
FIG. 2 is a schematic view of the fracture fixation system of FIG. 1.
FIG. 3 is a transparent perspective view of the fixation system of FIG. 1.
FIG. 4 is a side view along direction A of the fixation element of FIG. 3.
FIG. 5 is a front view of an assembly of a lag screw and compression nut of the fracture fixation system of FIG. 2.
FIG. 6 is a schematic view of the lag screw of FIG. 5.
FIG. 7 is a transparent perspective view of the compression nut of FIG. 5.
FIG. 8 is a top view of an assembly of an end connector coupled to first and second cap screws of the fracture fixation system of FIG. 2.
FIG. 9 is a schematic side view of the assembly of FIG. 8.
FIGS. 10-13 illustrate method steps of using the fracture fixation system of FIG. 1.
FIG. 13A is a schematic view of a double action screwdriver of FIG. 13 in use with the fracture fixation system of FIG. 2.
FIGS. 14A-14C are schematic views of a fracture fixation system according to another embodiment of the disclosure.
FIGS. 15A-15F illustrate method steps of using the fracture fixation system of FIGS. 14A-14C.
FIGS. 16A-16C are schematic views of a fracture fixation system according to another embodiment of the disclosure.
FIGS. 17A-17B are schematic view of a fracture fixation system according to another embodiment of the disclosure.
FIG. 18 is a schematic view of a fracture fixation system according to another embodiment of the disclosure.
FIGS. 19A-19M illustrate method steps of using the fracture fixation system of FIG. 18.

### DETAILED DESCRIPTION

The present disclosure describes a fracture fixation system, preferably for a femoral fracture (e.g., femoral neck fractures) used particularly to mount and install on a femur to provide support for the femur and assist with healing of a proximal femoral fracture. The fixation device may include a barrel defining a passageway and adapted to receive lag screws. The lag screws are sized and shaped to fit within the passageway of the barrel and extend further distally through a distal opening in the barrel. The lag screws may be inserted into the passageway of the barrel without passing completely through the barrel, such that proximal ends of the lag screws are held or disposed within a portion of the barrel. A corresponding compression nut may be coupled to each respective lag screw to be used to bias the lag screws proximally and apply compressive pressure between the fractured bone portions within the femur. Cap screws may be applied to each respective compression nut and an end connector may be coupled to the cap screws to secure the lag screws and compression nuts within the barrel. It should be understood that the fracture fixation system described herein may be applied to long bones in general, and while directed to a femur in the present description, it may also be directed to a humerus, tibia and other long bones.

As used herein, the term "proximal," when used in connection with a device or components of a device, refers to the end of the device closer to the user of the device (e.g., surgeon or operator) when the device is being used as intended. On the other hand, the term "distal," when used in connection with a device or components of a device, refers to the end of the device farther away from the user (e.g., surgeon or operator) when the device is being used as intended. As used herein, the term "superior" refers to an upward direction on the page or relative to an anatomy of a person standing upright. On the other hand, the term "inferior" refers to a downward direction on the page or relative to an anatomy of a person standing upright. It should be understood that these terms are not limiting, but merely used for ease of description, and that varied orientations may cause directions to differ. As used herein, the terms "substantially, "generally," "approximately" and "about" are intended to mean that slight deviations from absolute are included within the scope of the term so modified.

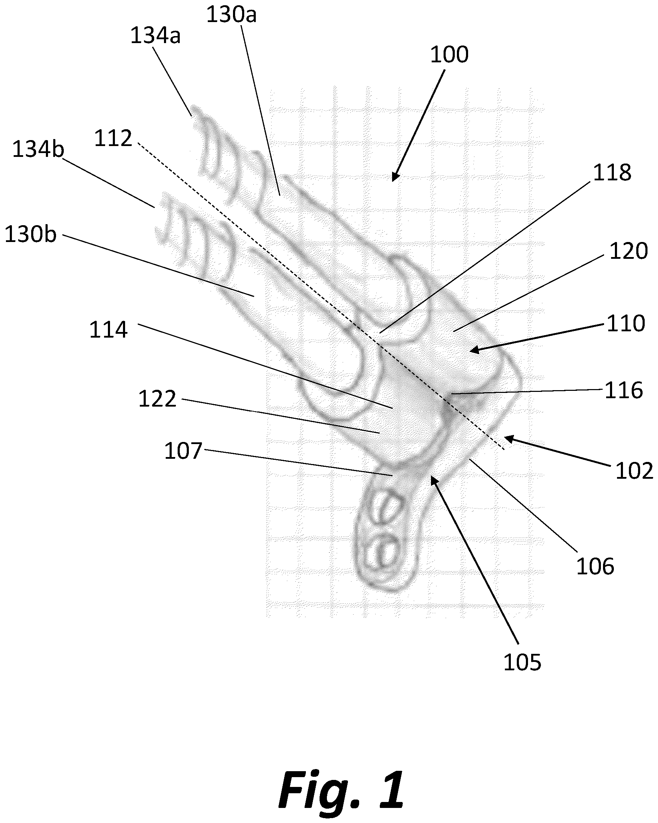

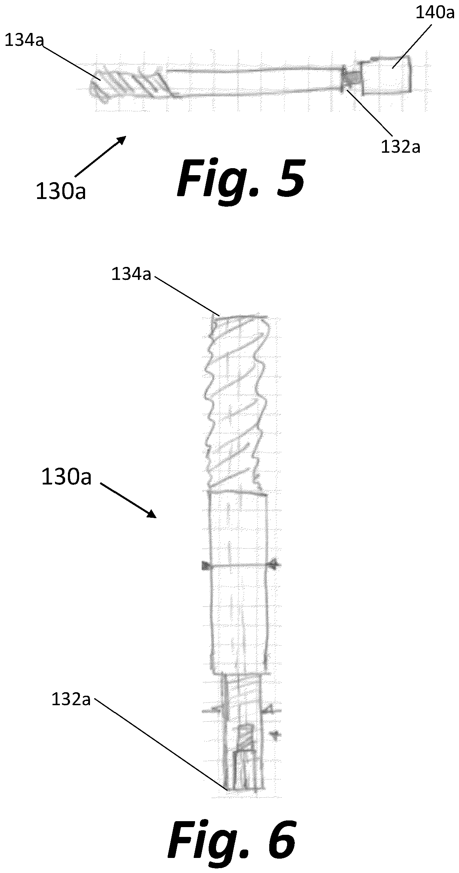

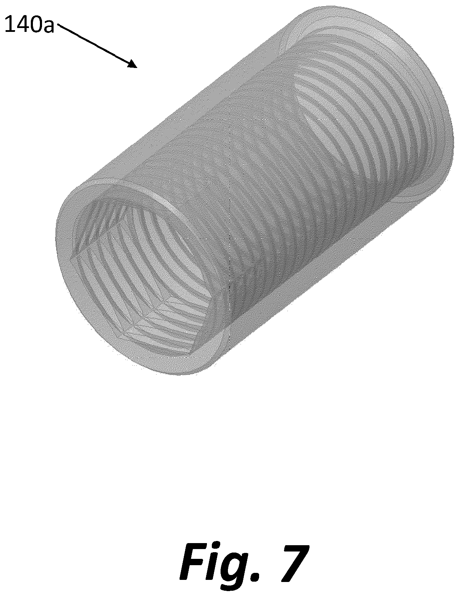

FIGS. 1-9 illustrate a fracture fixation system 100 according to an embodiment of the disclosure. The fracture fixation system 100 includes a fixation element 102 having a plate 105 defining an outer surface 106 and an inner surface 107 adapted for placement against an exterior of the femur when the fixation system 100 is in use. The fixation element 102 further includes a barrel 110 extending from a barrel proximal end 116 to a barrel distal end 118 along a barrel axis 112 and having a peripheral wall 114 that protrudes from the inner surface 107 of the plate 105. The barrel 110 may be manufactured based on patient-specific data to a particular size, shape and profile, which aids in effectively controlling varus forces.

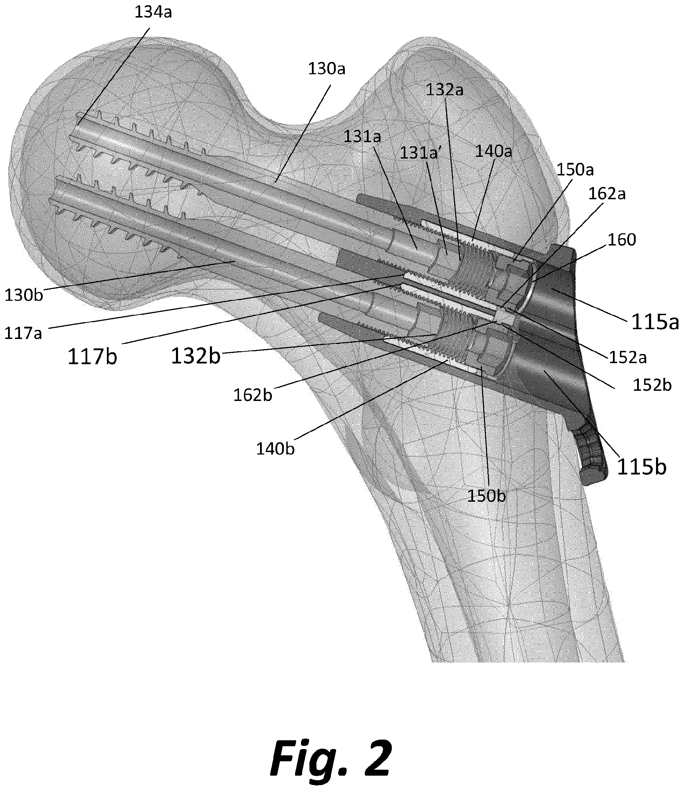

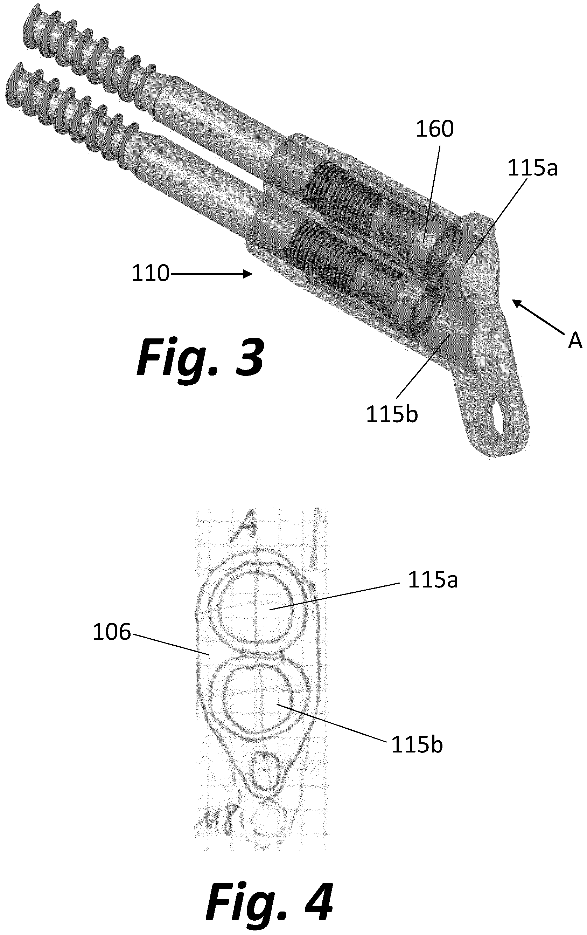

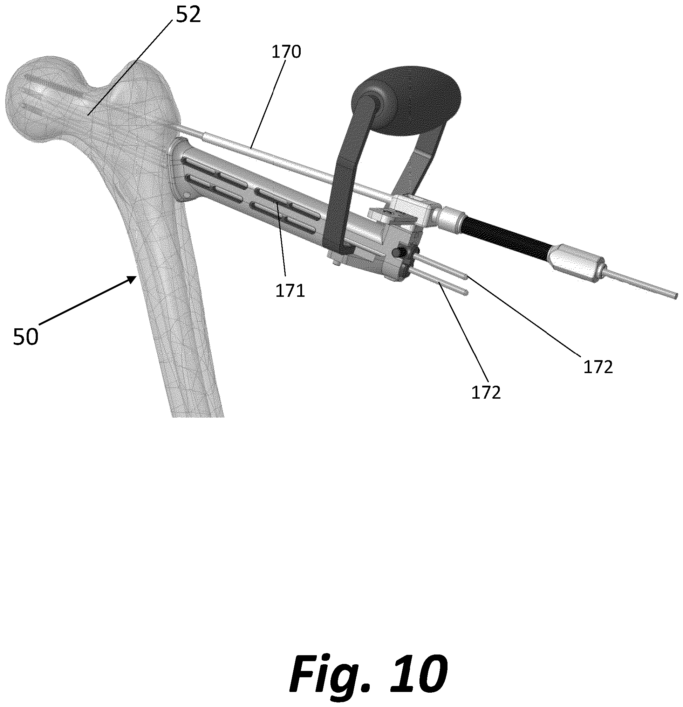

The fixation system 100 is used with a proximal femur 50 (shown in FIG. 10), such that in use, the barrel 110 extends towards and/or into a femoral neck 52. The barrel 110 includes a first superior cylindrical portion (or surface) 120 defining a first or superior passage 115a (shown in FIGS. 2-3) and a second inferior cylindrical portion (or surface) 122 defining a second or inferior passage 115b (FIG. 3), the cylindrical surfaces extending parallel to one another. The superior passage 115a and the inferior passage 115b also extend through corresponding portions of the plate 105, forming an upper lumen and a lower lumen through the fixation element 105. The superior cylindrical surface 120 and the inferior cylindrical surface 122 have approximately the same maximum diameter, but it is contemplated that the superior cylindrical surface may have a larger maximum diameter than the inferior cylindrical surface in some examples, and vice versa. Within each passage 115a, 115b, the internal diameter of the barrel 110 defines a corresponding shoulder 117a, 117b. That is, as each passage 115a, 115b extends distally, the internal diameter of each of the cylindrical surfaces 120, 122 transitions abruptly from a first diameter to a second diameter smaller than the first diameter, thereby forming the shoulder 117a, 117b within each respective passage 115a, 115b.

The fixation system 100 further includes a first or superior lag screw 130a extending from a proximal end 132a to a distal end 134a, the first lag screw 130a sized and shaped for insertion into the superior passage 115a. The fixation system 100 also includes a second or inferior lag screw 130b extending from a proximal end 132b to a distal end 134b, the second lag screw 130b sized and shaped for insertion into the inferior passage 115b. Each lag screw 130a, 130b has a threaded distal portion adjacent the respective distal end 134a, 134b which is inserted into the femur 50, and a proximal threaded portion adjacent the respective proximal end 132a, 134b. In the illustrated example, the lag screws 130a, 130b are identical in size and shape, however it is contemplated that they can vary in size and shape. For instance, the size of each lag screw may correspond to the size of the corresponding cylindrical portion and passage through which it extends. Thus, in examples in which the superior cylindrical portion is larger than the inferior cylindrical portion, the first lag screw may also be larger than the second lag screw in length, diameter, etc.

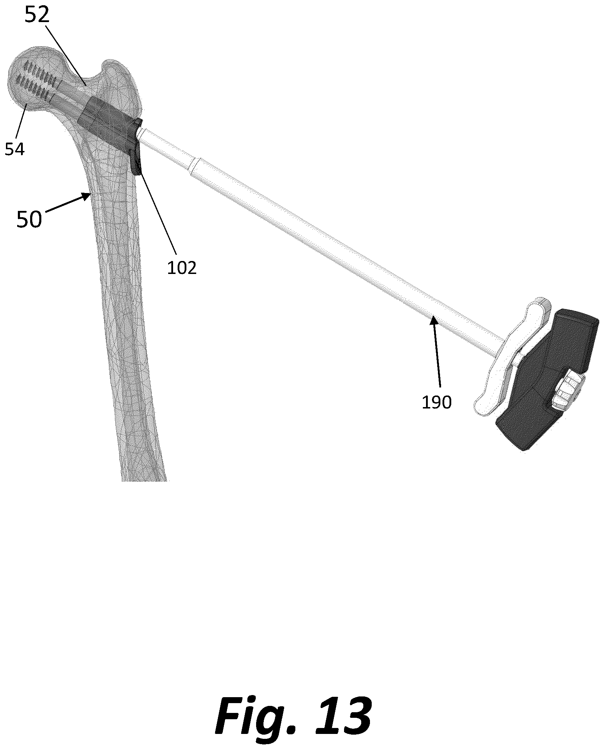

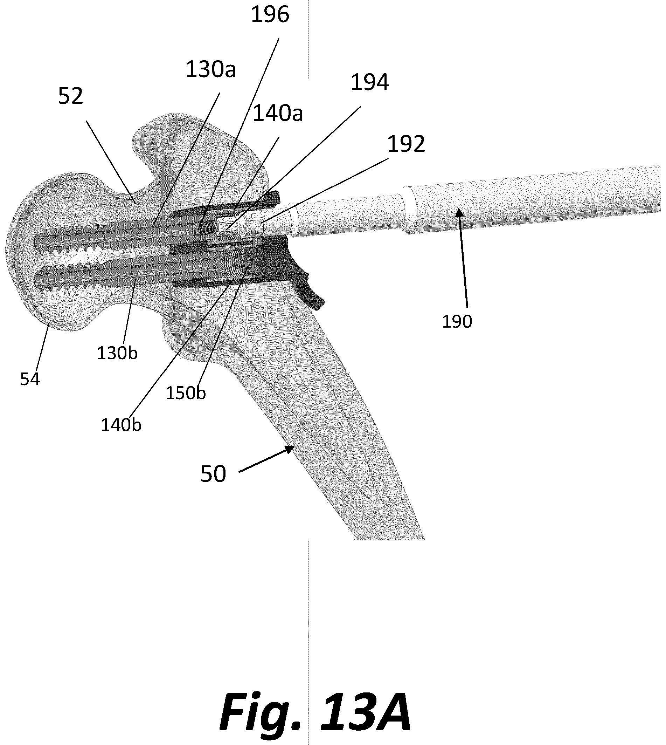

A first or superior compression nut 140a is disposed within the superior passage 115a and coupled to the proximal end 132a of the first lag screw 130a, and a second or inferior compression nut 140b is disposed within the inferior passage 115b and coupled to the proximal end 132b of the second lag screw 130b. Each compression nut 140a, 140b has an internally threaded surface threadably coupled to the proximal threaded portion of each respective lag screw 130a, 130b. The distal end of each compression nut 140a, 140b abuts and engages with the corresponding shoulder 117a, 117b to prevent or limit distal movement of the compression nuts 140a, 140b within the respective passages 115a, 115b. In some examples, the first lag screw 130a may be pre-assembled to the first compression nut 140a and the second lag screw 130b may be pre-assembled to the second compression nut 140b. A proximal internal portion of the compression nuts 140a, 140b define a shaped recess adapted to receive a tool (e.g., double action screwdriver 190 shown in FIG. 13A and discussed further below) to be engaged, such as a hex shape, to be rotated relative to the corresponding lag screw 130a, 130b as discussed further below. Although compression nuts 140a, 140b define a hex-shaped recess in the illustrated embodiment, any shape is contemplated.

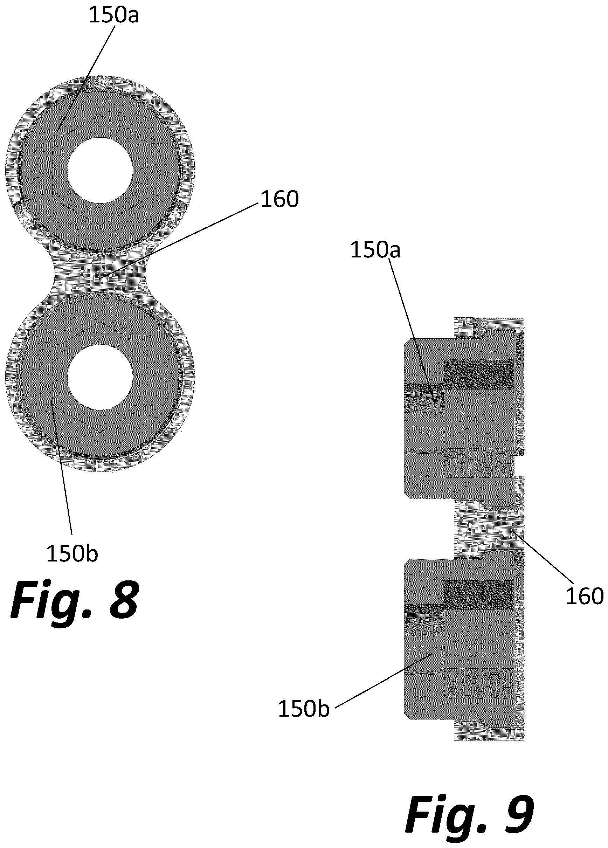

The fixation system 100 further includes a first or superior cap screw 150a disposed within the superior passage 115a and coupled to the proximal end of the first compression nut 140a and a second or inferior cap screw 150b disposed within the inferior passage 115b and coupled to the proximal end of the second compression nut 150b. Each cap screw 150a, 150b has a distal portion defining an externally threaded surface which engages with the internal threaded surface of each corresponding compression nut 140a, 140b. Each cap screw 150a, 150b defines a proximal engagement recess adapted to receive a tool to rotate the cap screws 150a, 150b relative to the respective compression nuts 140a, 140b, such as a hex shape as shown in FIG. 8. Each cap screw 150a, 150b defines a proximal lip 152a, 152b, respectively, as shown in FIG. 2. That is, as each cap screw 150a, 150b extends distally, the outer diameter of each cap screw 150a, 150b transitions abruptly from a first outer diameter to a second outer diameter smaller than the first outer diameter (e.g., such that the first outer diameter extends radially outward relative to the second diameter), thereby forming the proximal lip 152a, 152b. An end connector 160 is disposed within both the superior and inferior passages 115a, 115b and coupled to the first and second cap screws 150a, 150b by engaging with the proximal lip 152a of the first cap screw 150a and the proximal lip 152b of the second cap screw 150b. The end connector 160 has a pair of distal lips 162a, 162b disposed distally relative to the proximal lips 152a, 152b of the cap screws 150a, 150b. The end connector 160 is shaped to complementarily fit and couple to the cap screws 150a, 150b, and in some examples, the end connector 160 may be formed pre-assembled to the cap screws 150a, 150b.

It is contemplated that the fracture fixation system 100 may be packaged as a kit. The kit may include the plate 105 and barrel 110 formed as a single component, e.g., the fixation element 102, along with two lag screws 130a, 130b, two compression nuts 140a, 140b, two cap screws 150a, 150b and an end connector 160. The two lag screws 130a, 130b may be identical and shape and size, the two compression nuts 140a, 140b may be identical in shape and size, the two cap screws 150a, 150b may be identical in shape and size, and the end connector 160 may be symmetric such that the portion that engages with the first cap screw 150a is the same size as the portion that engages with the second cap screw 150b. Alternatively, one lag screw may be sized differently than the other, one compression nut may be sized differently than the other, one cap screw may be sized differently than the other, and one portion of the end connector may be sized differently than the other portion. It should be noted that the size of the lag screw may determine the size of each of compression nut, cap screw and portion of the end connector which correspond to that lag screw. As noted above, the first lag screw 130a may be pre-assembled to the first compression nut 140a and the second lag screw 130b may be pre-assembled to the second compression nut 140b in the kit. As also noted above, the first cap screw 150a and the second cap screw 150b may be pre-assembled to the end connector 160 in the kit.

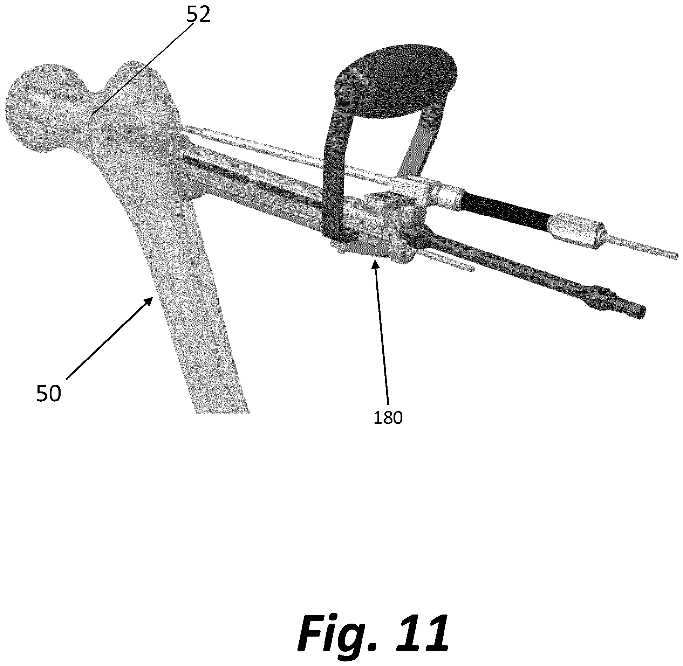

A method of using the fracture fixation system 100 is described with reference to FIGS. 10-14. First, a guide 171 is applied to the surface of the femur 50 to prepare the bone as shown in FIG. 10. The guide 171 may optionally be held in place against the surface of the femur 50 by a temporary fixator 170, which may later be removed after the guide is no longer needed. K-wire or pins 172 may be inserted through the guide and through the target areas of the femur 50 through which bores will be reamed and drilled for the fixation system 100. A first pair of bores is reamed, as shown by the barrel reamer 180 in FIG. 11, and drilled into the femoral neck. The first pair of bores is sized and shaped to receive the superior cylindrical portion 120 and the inferior cylindrical portion 122 of the barrel 110. These bores may overlap as needed to mirror the shape of the outer perimeter of the barrel 110. The first pair of bores includes a first superior bore having a first diameter, the first superior bore being drilled to a first depth in the femoral neck. The first pair of bores further includes a first inferior bore having a second diameter, the first inferior bore also being drilled to approximately to the first depth. In the illustrated example, the first superior bore and the first inferior bore have the same diameter and are drilled to the same depth, however it is contemplated that the diameters and the depths of such bores may vary, particularly when the respective sizes of the first cylindrical portion 120 and the second cylindrical portion 122 of the fixation element 102 vary.

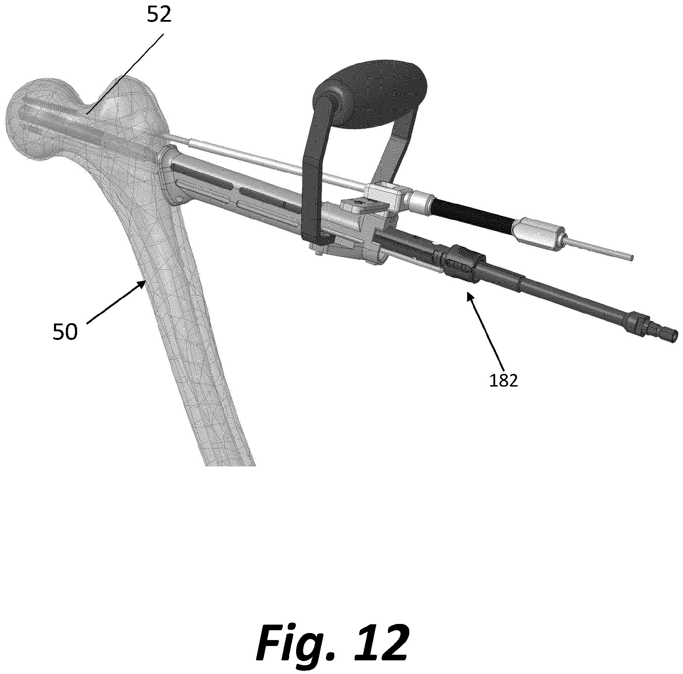

After the first pair of bores is drilled, a second pair of bores is reamed, as shown by the lag screw reamer 182 shown in FIG. 12, and drilled into the femoral neck. The guide 171 may also be used for these steps. The second pair of bores is sized and shaped to receive at least a portion of the first and second lag screws 130a, 130b, respectively. The second pair of bores includes a second superior bore drilled within the diameter of the first superior bore to a depth greater than the depth of the first superior bore. The second pair of bores further includes a second inferior bored drilled within the diameter of the second inferior bore to a depth greater than the depth of the first inferior bore. That is, the second superior bore has a diameter smaller than the first superior bore and a depth greater than the first superior bore, and the second inferior bore has a diameter smaller than the first inferior bore and a depth greater than the first inferior bore. The K-wire 172 is used as a guide for the reamers and drills to follow.

After the bores are drilled, the fixation element 102 is applied or mounted to the femur 50 such that the superior cylindrical portion 120 and the inferior cylindrical portion 122 are inserted into the first superior bore and the first inferior bore, respectively, and the inner surface 107 of the plate 105 abuts an exterior surface of the femur 50. The fixation element 102 may be placed using the K-wire 172 as a guide, for example, advancing the passages 115a, 115b along the K-wire 172.

The first lag screw 130a pre-assembled to the first compression nut 140a is then inserted into the superior cylindrical portion 120 such that the distal end 134a of the first lag screw 130a extends into the second superior bore and into the neck 52 and head 54 of the femur 50 beyond the fracture in the femur. The first lag screw 130a may be inserted by placing the lag screw 130a into or up against the superior passage 115a and engaging a tool, such as a hex-shaped tool (e.g., double-action screwdriver 190 shown in FIGS. 13 and 13A), with the proximal end of the first lag screw 130a to rotate and simultaneously distally translate the lag screw 130a through the passage 115a and into the second superior bore. Double-action screwdriver 190 includes a proximal hex 192, a distal hex 194 and a threaded distal tip 196. Threaded distal tip 196 is sized and shaped to be inserted into an internal threaded portion 131a of superior lag screw 130a as shown in FIG. 2 to axially fix the screwdriver 190 to the lag screw 130a. While distal threaded tip 196 is engaged with threaded portion 131a, distal hex 194 is sized, shaped and positioned to engage with a hex recess 131a' defined by superior lag screw 130a as shown in FIG. 2. Distal hex 194 rotationally couples screwdriver 190 to lag screw 130a, thereby allowing a surgeon to rotate lag screw 130a via rotation of screwdriver 190. In some scenarios, while distal threaded tip 196 and distal hex 194 are coupled to superior lag screw 130a, proximal hex 192 is sized and shaped such that it may be engaged with a hex-shaped recess of superior compression nut 140a, such that superior lag screw 130a and superior compression nut 140a may be rotated simultaneously. In other examples, double-action screwdriver 190 may be adjusted so that lag screw 130a and compression nut 140a may be rotated independently of one another. That is, screwdriver 190 may have a first (e.g., fixed) position in which distal hex 194 and proximal hex 192 engage lag screw 130a and compression nut 140a, respectively, to be rotated simultaneously, and screwdriver 190 may have a second (e.g., released) position which allows a user to engage only one of the lag screw 130a or the compression nut 140a to be rotated independently from the other.

The second lag screw 130b pre-assembled to the second compression nut 140b is then inserted into the inferior cylindrical portion 122 such that the distal end 134b of the second lag screw 130b extends into the second inferior bore and into the neck 52 and head 54 of the femur 50 beyond a fracture of the femur 50. The second lag screw 130b may be inserted in generally the same manner as the first lag screw 130a as described above. In some examples, the compression nuts 140a, 140b may not be pre-assembled to the lag screws 130a, 130b and may need to be threadably coupled to the proximal ends 134a, 134b of the lag screws 130a, 130b. As the lag screws 130a, 103b may be cannulated as shown in FIG. 2, the lag screws 130a, 130b may be implanted with the support of the K-wire 172, e.g., advancing a lumen of the lag screw along the K-wire 172.

After the lag screws 130a, 130b and compression nuts 140a, 140b are implanted such that the distal ends of the compression nuts 140a, 140b abut the corresponding shoulders 117a, 117b of the barrel 110, the compression nuts 140a, 140b are rotated to draw the corresponding lag screw 130a, 130b proximally. The compression nuts 140a, 140, may be rotated independently using an individual driving tool, or simultaneously using, e.g., double-action screwdriver 190 as shown in FIGS. 13 and 13A. As described above, double-action screwdriver 190 includes two driving heads, one of which (proximal hex 196) is able to engage with the engagement recess of each compression nut 140a, 140b, e.g., the hex bore defined by each compression nut 140a, 140b. Double-action screwdriver 190 may then either simultaneously or independently actuate a compression nut (e.g., 140a) and a lag screw (e.g., 130a). As the first compression nut 140a is rotated, the first compression nut 140a may remain in its axial position with respect to the barrel axis 112 abutting shoulder 117a within the superior passage 115a and cause the first lag screw 130a to translate proximally relative to the first compression nut 140a to compress the fractured pieces of the femur (i.e., the engaged distal portion of the femur) due to the threaded relationship between the compression nut 140a and the lag screw 130a. Similarly, as the second compression nut 140b is rotated, the second compression nut 140b remains in its axial position with respect to the barrel axis 112 abutting the shoulder 117b within the inferior passage 115b and causes the second lag screw 130b to translate proximally relative to the second compression nut 140b to compress the fractured piece of the femur due to the threaded relationship between the second compression nut 140b and the second lag screw 130a.

The first cap screw 150a is then threadably coupled to the proximal end of the first compression nut 140a and the second cap screw 150b is threadably coupled to the second compression nut 140b using a tool which engages with the engagement portion (e.g., hex bores) of the cap screws 150a, 150b. In the illustrated example, the end connector 160 is pre-assembled to the cap screws 150a, 150b, and thus they are installed together as a unitary component. That is, the assembly of the cap screws 150a, 150b are the end connector 160 are positioned into and/or over the passages 115a, 115b of the barrel 110 conjunctively and the cap screws 150a, 150b are then coupled to, e.g., by screwing, the corresponding compression nuts 140a, 140b. In other examples, the end connector 160 may be positioned over or into the passages 115a, 115b of the barrel 110 prior to the cap screws 150a, 150b, and the cap screws 150a, 150b may thereafter be placed in and actuated through the corresponding holes formed by the end connector 160.

It is contemplated that the above-described design could also be modified to include only one barrel and one lag screw assembly to be inserted therein. That is, the fracture fixation system may include a fixation element having a barrel defining only a single passage (rather than two passages), a single lag screw pre-assembled to a single compression nut, and a single cap screw pre-assembled to an end cap. Such a fixation system would be implanted in generally the same manner as described above.

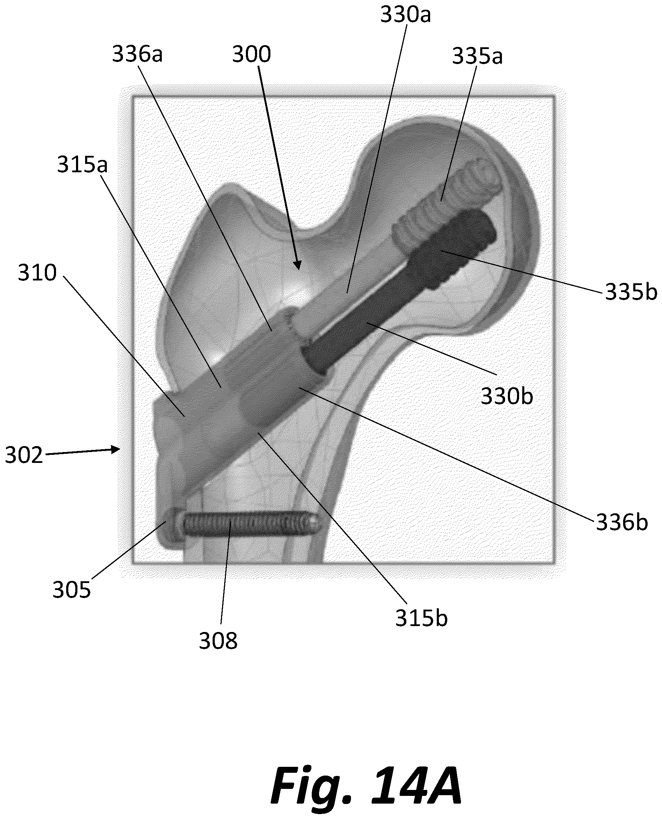

Whereas the fracture fixation system 100 disclosed above is described having superior lag screw 130a and inferior lag screw 130b, it is also contemplated that the fracture fixation system may be modified to replace at least one of the superior and inferior lag screws with alternative features for fixation. Several alternative embodiments are described below and illustrated in FIGS. 14A - 19I. Each of the embodiments described includes a barrel and plate as in the embodiments described above. It is contemplated that the elements coupled to and extending through the barrel, e.g., screw, peg, bolt, *etc.,* may be coupled to the barrel using the assembly described above where applicable, e.g., compressions nuts, cap screws, end connectors, *etc..* Alternatively, it is contemplated that the elements extending through the barrel may be coupled to the barrel by different means, as set forth in the respective descriptions below. It is noted that like numbers represent like elements throughout the description.

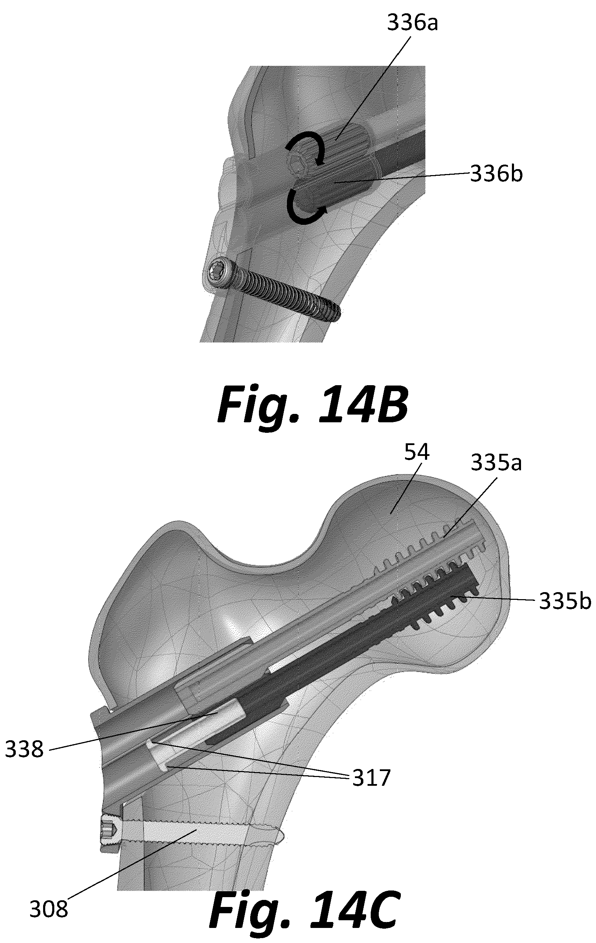

FIGS. 14A-14C illustrate a fracture fixation system 300 having a first lag screw or superior gear screw 330a and a second lag screw or inferior gear screw 330b disposed within and extending from barrel 310. Superior gear screw 330a includes a distal threaded portion 335a which is threaded in a first direction (e.g., a right-handed direction) and a proximal toothed portion 336a. Similarly, inferior gear screw 330b includes a distal threaded portion 335b threaded in a second direction opposite the first direction, (e.g., a left-handed direction) and a proximal toothed portion 336b. As shown in FIG. 14B, each of proximal toothed portions 336a, 336b include elongate teeth protruding radially from an inner radius of superior and inferior gear screws 330a, 330b, respectively, and extending generally parallel to a longitudinal axis of each of the superior and inferior gear screws. Barrel 330 is configured such that superior passage 315a is in communication with inferior passage 315b, thereby allowing proximal toothed portion 336a of superior gear screw 330a to interact and engage with proximal toothed portion 336b of inferior gear screw 330b. As shown more clearly in FIG. 14C, gear screws 330, 330b are sized and positioned such that distal threaded portion 335a of superior gear screw 330a overlaps with distal threaded portion 335b of inferior gear screw 330b to form a meshed contact. Due to the engagement of the opposing threads of gear screws 330a, 330b, the gear screws may be advanced into femoral head 54 by rotation of one gear screw in a first direction and rotation of the other gear screw in a second direction opposite the first direction. For example, superior gear screw 330a may be rotated in a clockwise direction and/or inferior gear screw 330b may be rotated in a counterclockwise direction. It is noted that rotation of only one of superior gear screw 330a or inferior gear screw 330b causes rotation of the other due to the engagement of the opposing threads through synchronized screw insertion. Fixation system 300 further includes compression screw 338 as described below which is used to secure the fracture fixation system within the bone, and fixation screw 308 inserted through plate 305 for further securement to the bone.

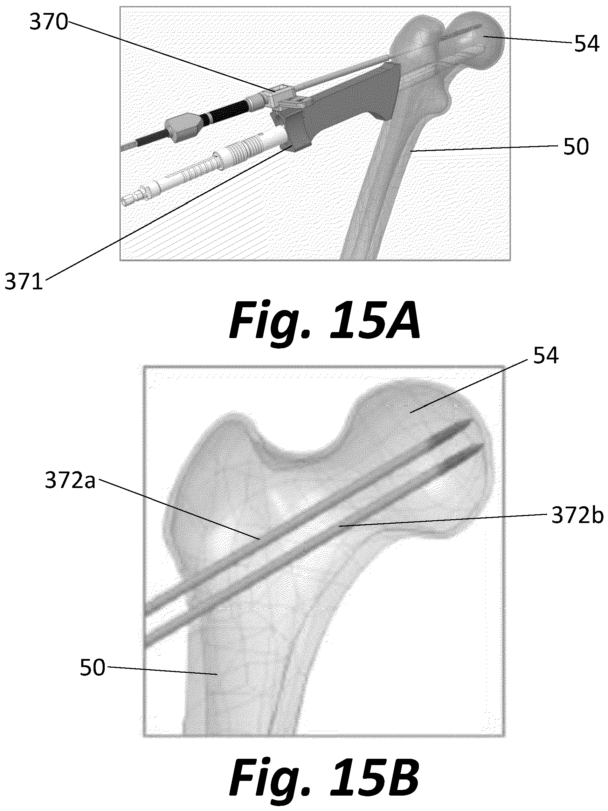

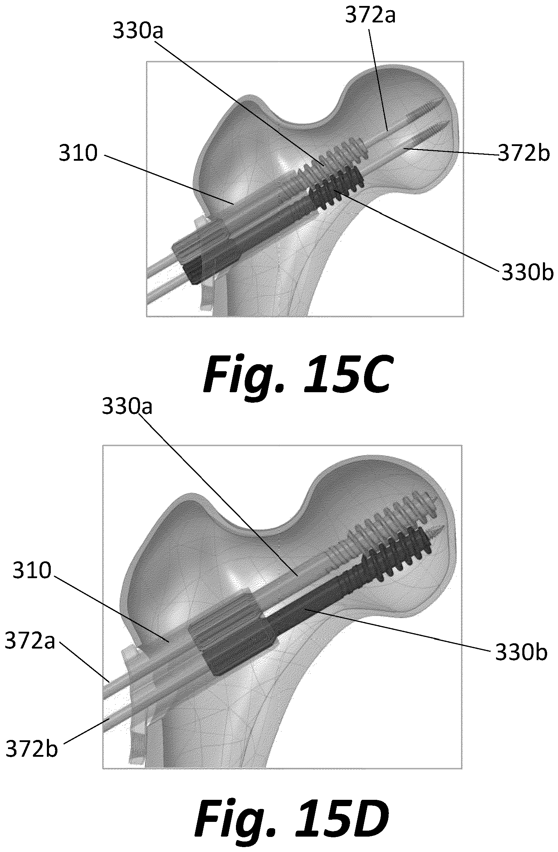

A method for installing and inserting fracture fixation system 300 is shown in FIGS. 15A-15F. Initial holes may be drilled into femur 50 and femoral head 54 as shown in FIG. 15A using aiming guide 371 with temporary fixator 370. In some examples, superior guide pin 372a (or K-wire) and inferior guide pin 372b (or K-wire) shown in FIG. 15B may be used to ream and drill the holes into the femur 50. Guide pins 372a, 372b may further act as a guide for both fixation plate 302 and gear screws 330a, 330b to be advanced therealong as described below.

Barrel 310 may be inserted into the femur prior to insertion of gear screws 330a, 330b therein or alternatively with the gear screws already at least partially disposed within barrel 310. That is, in some examples, barrel 310 may be installed onto the femur 50 first, and gear screws 330a, 330b may be inserted through barrel 310 and into the femur 50 thereafter. In other examples, barrel 310 may be applied to the femur 50 with gear screws 330a, 330b at least partially inserted within barrel 310 such that distal threaded portions 335a, 335b are disposed within or extending distally from barrel 310, and the gear screws may be further advanced into the femoral head thereafter if further advancement is needed. Superior gear screw 330a may be advanced along superior guide pin 372a and inferior guide screw 330b may be advanced along inferior guide pin 372b as shown in FIGS. 15C-15D.

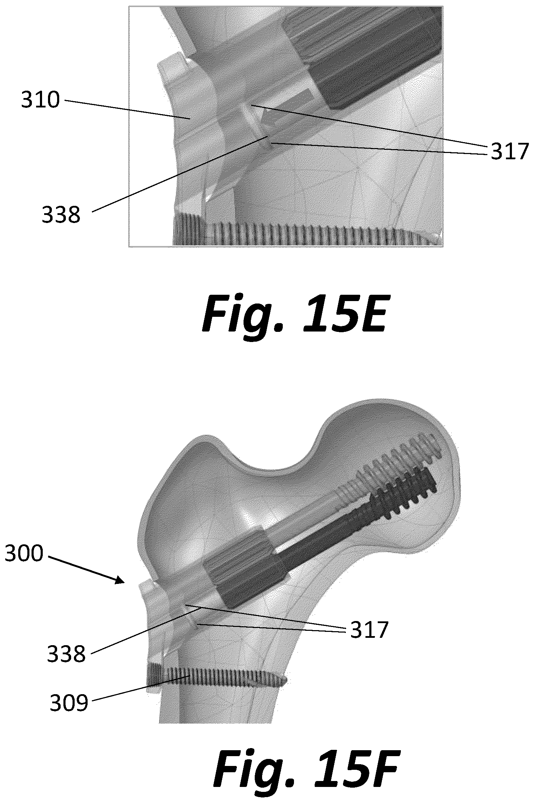

After positioning of fixation element 302 and gear screws 330a, 330b onto and into femur 50, the gear screws may be rotated to compress the femoral fracture as described above. In some examples, rotation may be performed with a rotation tool and/or component such as compression screw 338 shown in FIGS. 14C and 15E-15F. In some examples, a distal end of compression screw 338 has an external threading sized to engage with an internal threading of an interior bore defined by inferior gear screw 330b. A rotation tool, such as a screwdriver, may then be inserted into barrel 310 to engage with and rotate compression screw 338 relative to inferior gear screw 330b. Rotation of compression screw 338 relative to inferior gear screw 330b will create relative axial movement between the two components, such as axial or distal advancement of compression screw 338 relative to inferior gear screw 330b. Compression screw 338 may be advanced distally through barrel 310 until a head of compression screw 338, which has a greater diameter than the body of the compression screw, reaches and abuts a shoulder 317 formed in the interior of barrel 310. Shoulder 317 is formed by an abrupt change in the internal diameter of barrel 310, whereby the barrel has a first internal diameter near its proximal end which is greater than a second internal diameter near its distal end. The second internal diameter is less than the diameter of the head of compression screw 338, thereby preventing the head of compression screw 338 from advancing beyond shoulder 317. Once the head of compression screw 338 abuts shoulder 317, compression screw 338 may be further rotated to draw inferior gear screw 330b proximally with respect to barrel 310, which will also draw superior gear screw 330a proximally due to the engagement of superior and inferior gear screws 330a, 330b, and thus create compressional forces along the femoral fracture.

Although not shown, it is contemplated that superior gear screw 330a may be engaged by a compression screw instead of inferior gear screw 330b. It is also contemplated that both superior and inferior gear screws 330a, 330b may be engaged simultaneously with compression screws to apply balanced forces to both gear screws. A fixation screw 309 may be inserted and passed through plate 305 at any time after fixation element 302 is applied to femur 50 to further secure fixation element 302 in place on the bone. Fully implanted fracture fixation system 300 with fixation screw 309 is shown in FIG. 15F. Compression screw 338 may remain disposed within barrel 310 and coupled to inferior gear screw 330b in some examples, and may be removed from barrel 310 in other examples.

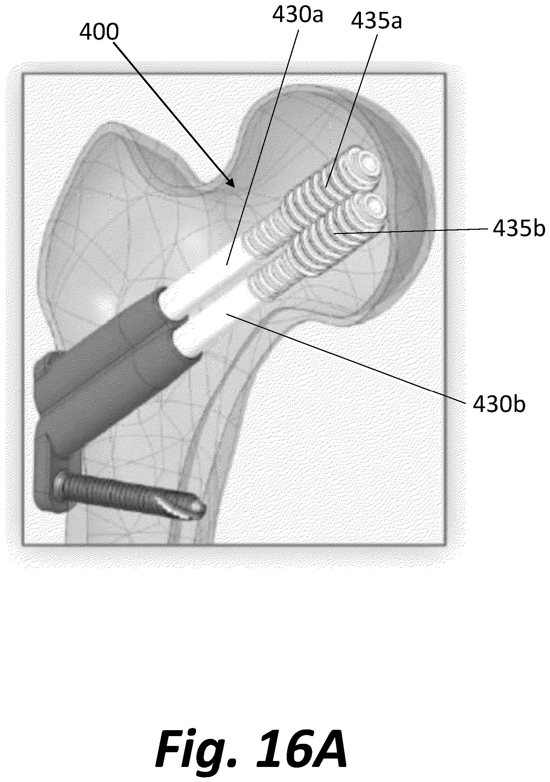

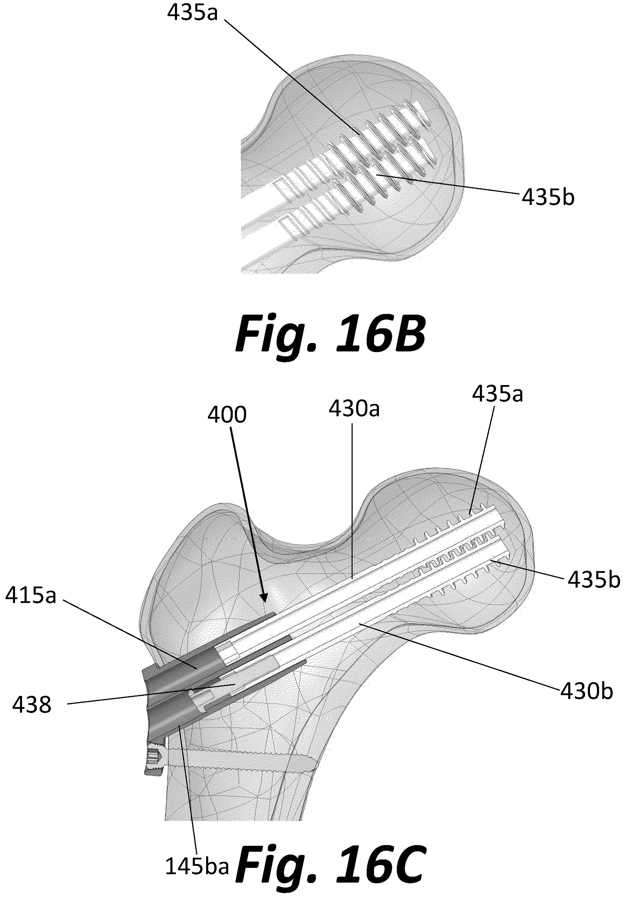

Fracture fixation system 400 shown in FIGS. 16A-16C is substantially similar to fixation system 300. Fixation system 400 includes a first lag screw or superior link screw 430a having distal threaded portion 435a and a second lag screw or inferior link screw 430b having distal threaded portion 435b. Distal threaded portion 435a of superior link screw 430a is threaded in a first direction and distal threaded portion 435b of inferior link screw 430b is threaded in the same direction. For example, both distal threaded portions 435a, 435b may be threaded in a right-handed direction, or both portions may be threaded in a left-handed direction. Link screws 430a, 430b are sized and positioned such that distal threaded portions 435a, 435b overlap and engage with each other, similar to distal threaded portions 335a, 335b described above. Link screws 430a, 430b do not include proximal tooth portions, but instead may be separated within barrel 410 into superior passage 415a and inferior passage 415b, respectively, by a central barrel wall such that proximal portions of the links screws do not interact or engage with each other. Link screws 430a, 430b may be actuated in generally the same manner as described above with respect to gear screws 330a, 330b, e.g., with compression screw 438.

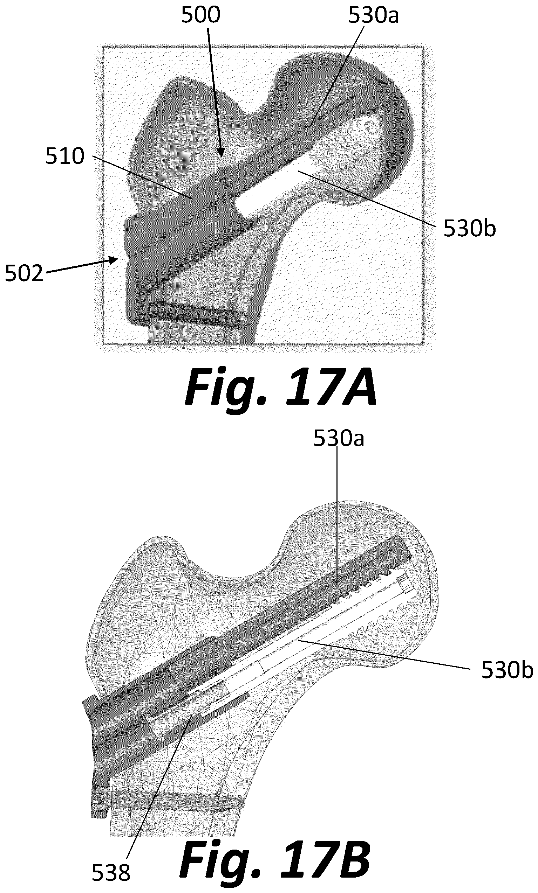

As shown in FIGS. 17A-17B, fracture fixation system 500 includes a superior bolt 530a and an inferior lag screw 530b disposed within and extending from fixation element 502. Inferior lag screw 530b is substantially similar to inferior lag screw 130b. Superior bolt 530a is an elongate component having a generally consistent non-circular cross-section along the length of the bolt. Superior bolt 530a may, for example, have a cross-section in the shape of a triangle, rectangle, star or the like in order to resist rotation of fixation element 502 within femur 50. Inferior lag screw 530b may be rotated in a manner similar to that described above (e.g., with use of compressions crew 538) to increase the compressive force on the femoral fracture, while superior bolt 530a is shaped to resist rotation of barrel 510, for example, so that the orientation of fixation element 502 is substantially maintained particularly during rotation of compression screw 538 and/or inferior lag screw 530b.

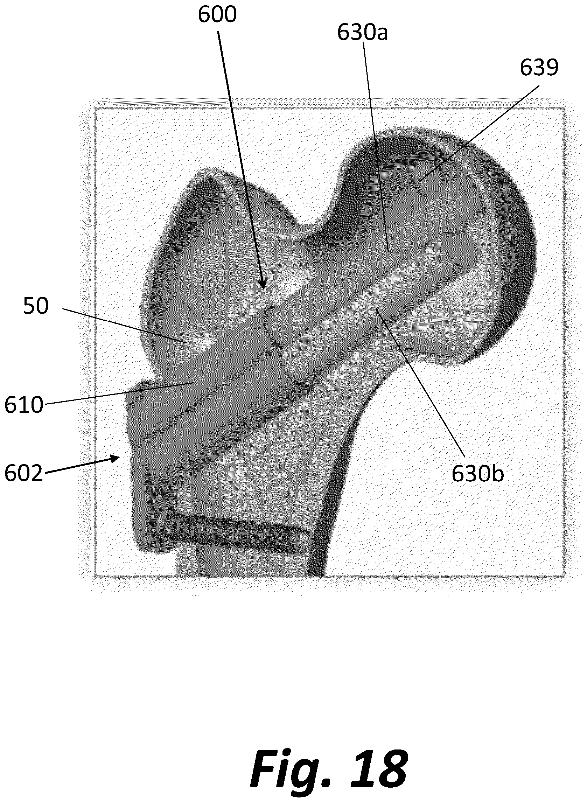

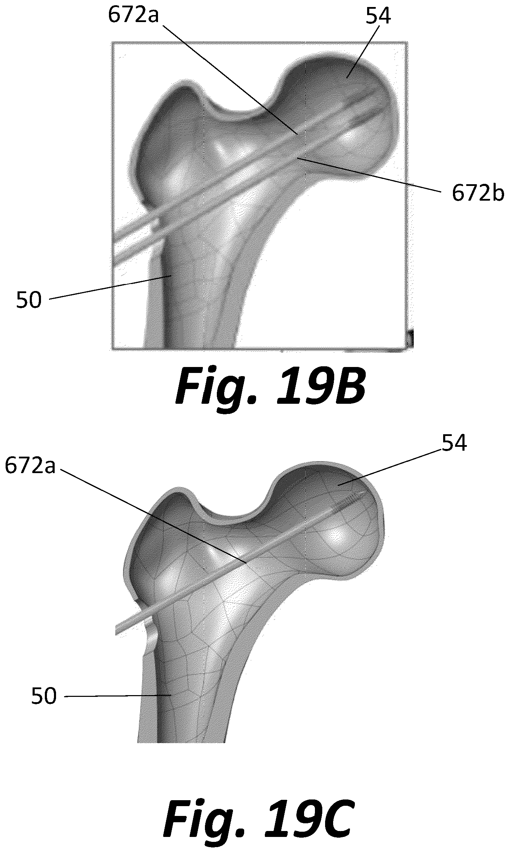

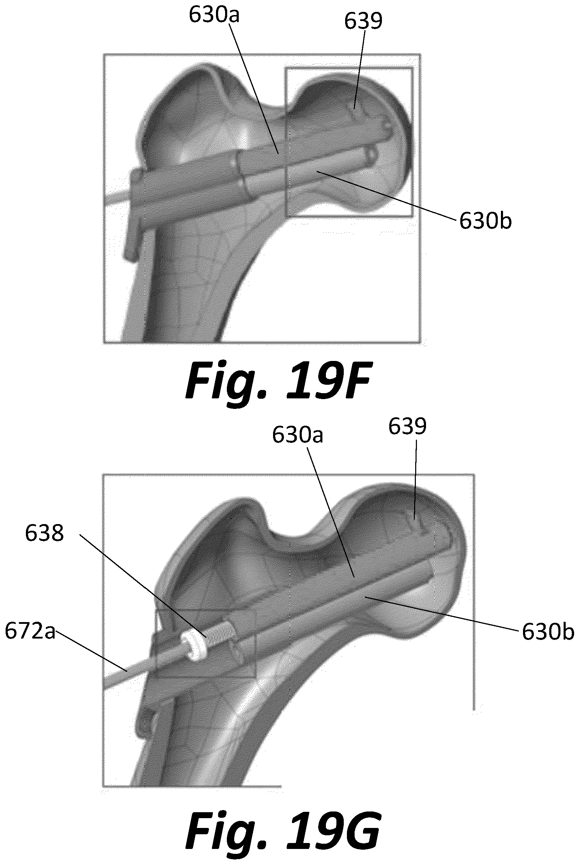

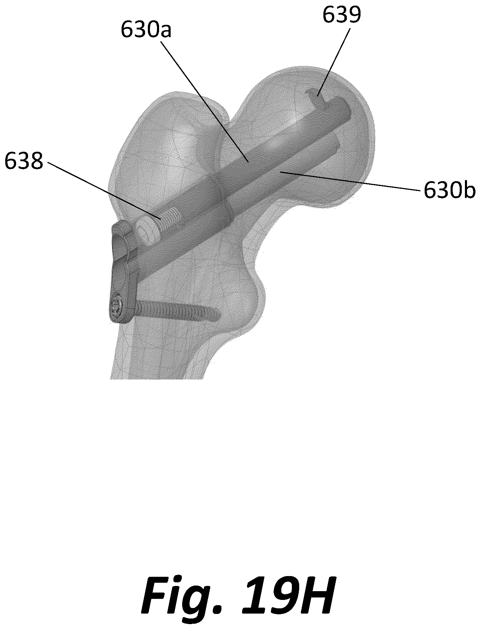

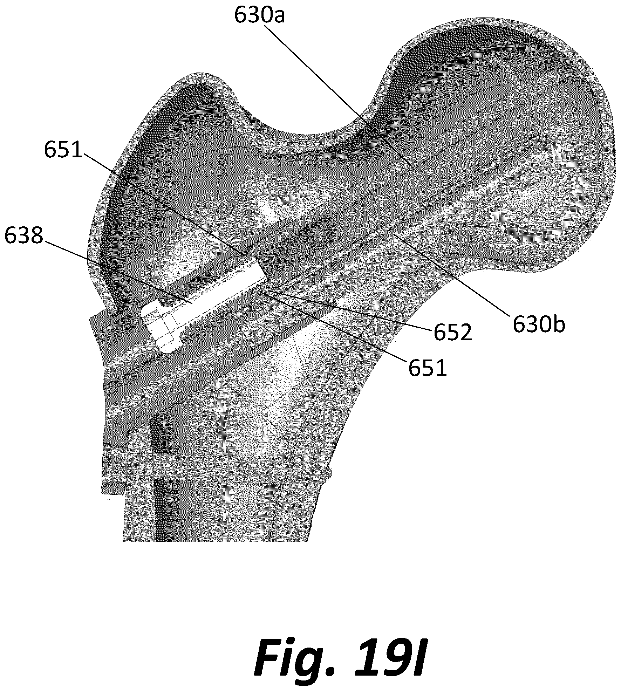

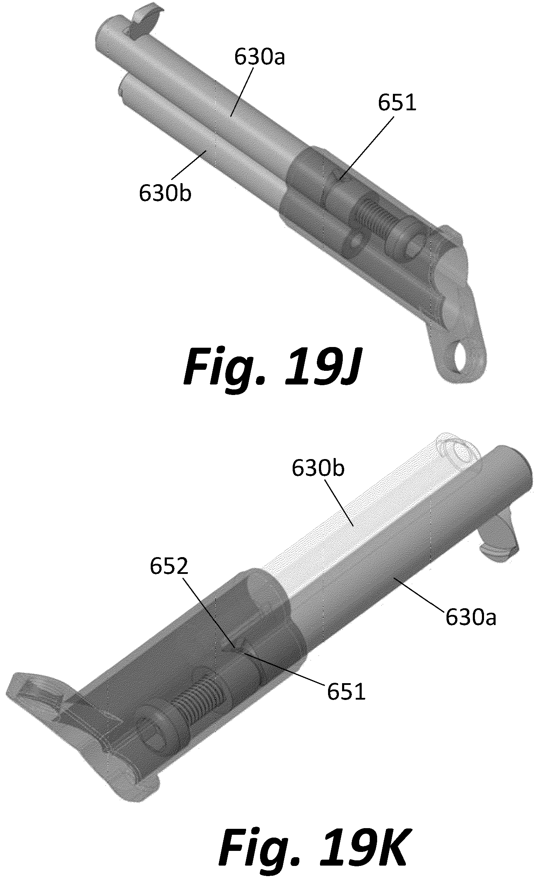

As shown in FIG. 18, fracture fixation system 600 has a first or superior bolt 630a and a second or inferior bolt 630b. Superior bolt 630a is elongated in a longitudinal direction and has a generally circular rounded cross-section along its length, although other cross-sectional shapes are contemplated. Superior bolt 630a includes a blade 639 positioned proximate a distal end of the bolt, the blade extending generally orthogonal to the length of superior bolt 630a and having a proximal-pointing hook at a tip of the blade (shown more clearly in FIG. 19G). The positioning of blade 639 on superior bolt 630a and within femur 50 provides for fixation and active compression of fixation system 600 within femur 50 as well as intra- and postoperative rotational fixation of the bone fragment. Blade 639 is shown in a deployed configuration in FIG. 18, which reduces or prevents axial movement (e.g., retraction) of superior and inferior bolts 630a, 630b. Inferior bolt 630b is also elongated in a longitudinal direction parallel to that of superior bolt 630a, and inferior bolt 630b is generally cylindrical except for a recess defined along the length of bolt 630b which is configured for engagement with superior bolt 630a. In the implanted configuration of fixation system 600 shown in FIG. 18, the recess defined by inferior bolt 630b is positioned along a superior surface of inferior bolt 630b adjacent superior bolt 630a such that a portion of superior bolt 630a is received within the recess of inferior bolt 630b. The engagement and positioning of superior bolt 630a within the recess of inferior bolt 630b along with a protrusion 652 extending from a proximal portion of inferior bolt 630b and engaging with a recess 651 defined in a corresponding proximal portion of superior bolt 630a as shown in FIG. 19I (and described further below) allows for linked construct sliding of the bolts through barrel 610.

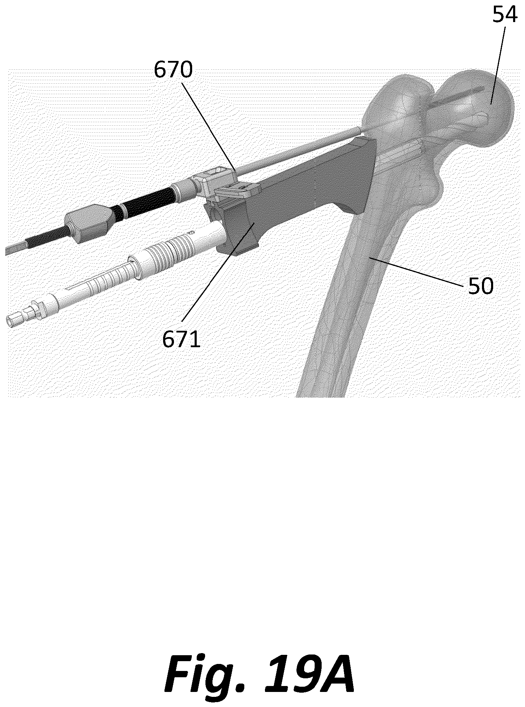

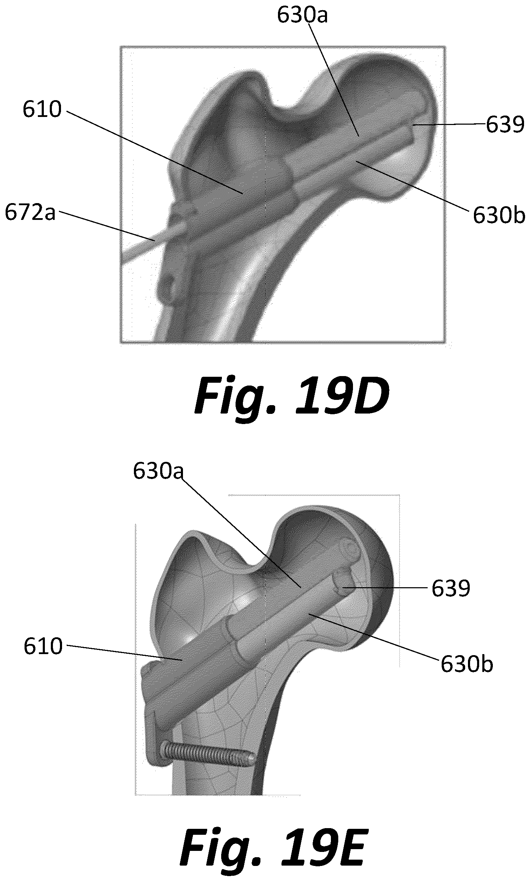

A method for inserting and installing fracturing fixation system 600 is shown in FIGS. 19A-19I. First, aiming device 671 may optionally be placed on lateral cortical bone of femur 50 with temporary fixator pin 670 extending into femoral head 54. Superior guide pin or K-wire 672a and inferior guide pin or K-wire 672b may be inserted and used as guides for reaming holes for barrel 610 and bolts 630a, 630b in the manner described above with reference to other embodiments. After holes are reamed, aiming device 671 and inferior K-wire 672b may be removed as shown in FIG. 19C, and fixation element 602 may be inserted over superior K-wire 672a such that superior bolt 630a and a superior portion of barrel 610 are advanced along superior K-wire 672a as shown in FIG. 19D. Superior bolt 630a may be engaged with inferior bolt 630b during insertion of the bolts. For instance, superior bolt 630a may be received within the recess defined by inferior bolt 630b. During insertion, blade 639 is in the undeployed configuration shown in FIGS. 19D and 19E wherein blade 639 is adjacent and abutting a distal end of inferior bolt 630b so that blade 639 does not interact with excess bone during insertion.

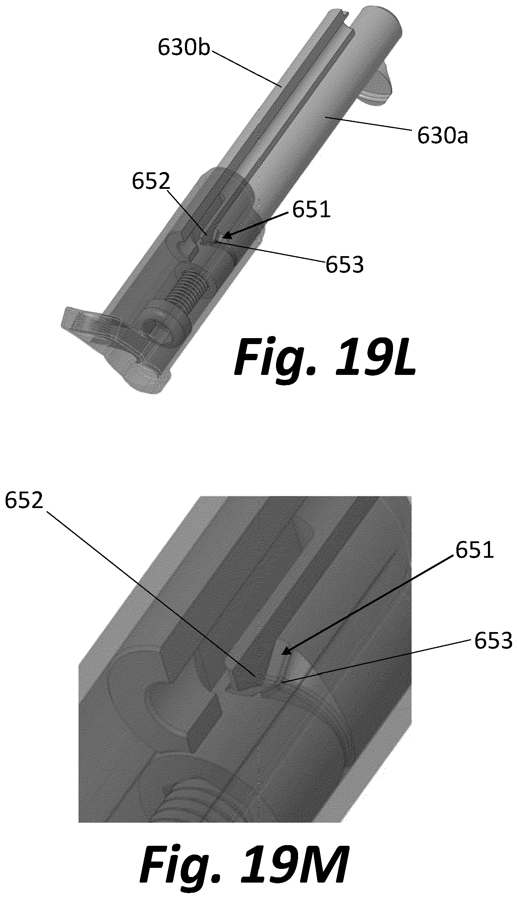

After insertion of superior and inferior bolts 630a, 630b, blade 639 may be deployed as shown in FIG. 19F by rotating superior bolt 630a using a rotation tool such as a screwdriver to engage and rotate a proximal end of superior bolt 630a. After deployment of blade 639, compression screw 638 may be advanced over superior K-wire 672a and inserted into a proximal end of superior bolt 630a to engage with and be rotated relative to superior bolt 630a as described in other embodiments herein (e.g., compression screw 338), thereby drawing superior and inferior bolts 630a, 630b in a proximal direction and increasing compressive forces on the femoral fracture. As shown in FIGS. 19I-19M, superior bolt 630a defines a circumferential recess 651 near a proximal end of the superior bolt, and inferior bolt 630b includes a protrusion 652 near a proximal end of the inferior bolt extending beyond the circumference of the inferior bolt to mate with circumferential recess 651 of superior bolt 630a. The mating of protrusion 652 and recess 651 maintains the relative axial positioning of superior bolt 630a and inferior bolt 630b as compression screw 638 is rotated so that both superior bolt 630a and inferior bolt 630b are drawn proximally together to simultaneously apply and increase compressive forces to the femoral fracture. Protrusion 652 can be disposed on a cantilever extension of inferior bolt 630b so that protrusion 652 can flex in and out of contact with recess 651. This also provides haptic feedback so that when superior bolt 630a is rotated, a user can feel when it locks into position. In the illustrated embodiment, circumferential recess 651 is only defined around half of the circumference of superior bolt 630a so that superior bolt 630a may only be transitioned from the undeployed configuration to the deployed configuration in a single direction (e.g., clockwise) and transitioned from the deployed configuration to the undeployed configuration in the reverse direction (e.g., counter-clockwise). Circumferential recess 651 also includes a lip 653, as shown in FIGS. 19L and 19M, which is adjacent the portion of the recess where the protrusion 652 is disposed when in the deployed configuration. Lip 653 provides haptic feedback to a user when the superior bolt 630a has been rotated approximately 180 degrees from the undeployed configuration and has reached the deployed configuration.

In some examples, fracture fixation system 600 may include a plurality of blades which may be disposed on and extend from the superior bolt, the inferior bolt, or both. The blade(s) may extend at a transverse angle from the superior or inferior bolts (e.g., extending straight from the bolt or forming a helix) such that in some examples, rotation of the bolt may cause proximal movement or force and result in compression upon rotation. The blade(s) may have a sharp leading (distal) edge to assist with penetration of the femur. The blade(s) may be formed with a sharp trailing (proximal) edge to assist with removal if necessary.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. A fracture fixation system, comprising:
a fixation element including a plate and a barrel, the plate having an outer surface and an inner surface for placement against an exterior surface of a bone, and the barrel extending along a barrel axis and having a peripheral wall protruding from the inner surface of the plate, the fixation element defining first and second passages through the plate and the barrel that each extend along the barrel axis;
a first lag screw or bolt extending from a proximal end to a distal end and configured to be inserted through at least the first passage defined by the plate and the barrel; and
a second lag screw or bolt extending from a proximal end to a distal end and configured to be inserted through the second passage defined by the plate and the barrel.

2. The fracture fixation system of claim 1, further comprising a first compression nut configured to be coupled to the proximal end of the first lag screw or bolt when the first lag screw or bolt is disposed within the first passage.

3. The fracture fixation system of claim 2, wherein the first compression nut defines an internally threaded surface that is configured to be threadably coupled to an externally threaded surface at the proximal end of the first lag screw or bolt.

4. The fracture fixation system of claim 2 or claim 3, wherein the barrel defines a first internal shoulder within the first passage, and a distal end of the first compression nut is configured to abut the first internal shoulder to limit distal movement of the first compression nut within the first passage.

5. The fracture fixation system of any one of claims 2-4, further comprising a first cap screw configured to be coupled to the internally threaded surface of the first compression nut.

6. The fracture fixation system of any one of claims 1-5, further comprising an end connector configured to be disposed within the first and second passages of the barrel.

7. The fracture fixation system of claim 1 or claim 6, further comprising a first compression nut configured to be coupled to the first lag screw or bolt and a second compression nut configured to be coupled to the second lag screw or bolt.

8. The fracture fixation system of claim 7, wherein the barrel defines a first internal shoulder within the first passage and a second internal shoulder within the second passage, and wherein a distal end of the first compression nut is configured to abut the first internal shoulder and a distal end of the second compression nut is configured to abut the second internal shoulder.

9. The fracture fixation system of any one of claims 1-8, wherein the first passage is separated from the second passage by a central portion of the barrel.

10. The fracture fixation system of any one of claims 1-9, wherein the first lag screw or bolt includes a distal threaded portion having threads oriented in a first rotational direction along a length of the distal threaded portion, and the second lag screw or bolt includes a distal threaded portion having threads oriented in a second direction opposite the first rotational direction along a length of the distal threaded portion of the second lag screw or bolt.

11. The fracture fixation system of claim 10, wherein the first lag screw or bolt includes a proximal portion having teeth configured to be disposed within the first passage, and wherein the second lag screw or bolt includes a proximal portion having teeth configured to be disposed within the second passage.

12. The fracture fixation system of claim 11, wherein the teeth of the first lag screw or bolt protrude from the perimeter of the proximal portion of the first lag screw or bolt and each tooth extends longitudinally along the proximal portion of the first lag screw or bolt, and wherein the teeth of the second lag screw or bolt protrude from the perimeter of the proximal portion of the second lag screw or bolt and each tooth extends longitudinally along the proximal portion of the second lag screw or bolt.

13. The fracture fixation system of any one of claims 1-9, wherein the first lag screw or bolt includes a distal threaded portion having threads oriented in a first rotational direction along a length of the distal threaded portion, and the second lag screw or bolt includes a distal threaded portion having threads oriented in the first rotational direction along a length of the distal threaded portion of the second lag screw or bolt.

14. The fracture fixation system of claim 13, wherein each thread of the first lag screw or bolt is configured to be positioned between a pair of adjacent threads on the distal threaded portion of the second lag screw or bolt.

15. The fracture fixation system of claim 1, wherein the first lag screw or bolt is a bolt and the second lag screw or bolt is a bolt, and wherein a distal portion of the first bolt includes a blade extending oblique to a longitudinal axis of the first bolt.
